# EUROPÄISCHE PATENTANMELDUNG

(11) **EP 1 997 378 A2**
(43) Veröffentlichungstag der Anmeldung: **03.12.2008**
(21) Anmeldenummer: 08007813.2
(22) Anmeldetag: 23.04.2008
(51) Int. Cl.: A01N 43/16, A01N 65/00, A01N 37/46, A01N 25/04, A61K 8/49, A61K 8/97, A61Q 17/02, A01P 17/00

(54) **Repellentien gegen Wespen**

(30) Priorität: 31.05.2007 DE 102007026049
(71) Anmelder: Beiersdorf AG, 20245 Hamburg (DE)
(72) Erfinder: Kröpke, Rainer, 22869 Schenefeld (DE); Nielsen, Jens, 24558 Henstedt-Ulzburg (DE); von der Fecht, Stephanie, 22880 Wedel (DE); Schulz, Jens, Dr., 25462 Rellingen (DE)
(74) Vertreter: Hartmann, Jost

(57) **Zusammenfassung**

Zubereitung umfassend 3-(N-n-Butyl-N-acetyl-amino)propionsäureethylester, Dihydronepetalacton und/oder ein Extrakt aus der Katzenminze sowie ein oder mehrere Verbindungen gewählt aus der Gruppe der Parfuminheltsstoffe als Wespenabwehrmittel.

## Beschreibung

Die vorliegende Erfindung betrifft eine Zubereitung umfassend 3-(N-n-Butyl-N-acetyl-amino)-propionsäureethylester, Dihydronepetalacton und/oder ein Extrakt aus der Katzenminze sowie ein oder mehrere Verbindungen gewählt aus der Gruppe der Parfuminhaltsstoffe 1,3,4,6,7,8-Hexahydro-4,6,6,7,8,8-hexamethylcyclopenta-gamma-2-benzopyran, 2,6-Dimethyl-7-octen-2-ol, 2-Aoetonapthone-1,2,3,4,5,6,7,8-octahydro-2,3,8,8-tetramethyl, 2-Isobutyl-4-hydroxy-4-methyltetrahydropyran, 2-tert-Pentylcyclohexylacetat, 3,7-Dimethyl-2,6-octadien-1-ol, 3-Methyl-5-phenyl-1-pentanol, 7-Acetyl-1,1,3,4,4,6-hexamethyltetralin, Adipinsäurediester, alpha-Amylcinnamaldehyd, alpha-Hexylcinnamaldehyde, alpha-Isomethylionon, Alpha-Methylionon, Amyl C Butylphenylmethylpropionalcinnamal, Amylcinnamylalkohol, Amylsalicylat, Anisalkohol, Benzoin, Benzylacetat, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citral, Citronellol, Citronellylmethylcrotonat, Citronenöl, Coumarin, Diethylsuccinat, d-Limonene, Ethylenbrassylate, Ethyllinalool, Eugenol, Evernia Furfuracea Extract, Evernia Prunastri Extract, Farnesol, Geraniol, Guajakholzöl, Heliotropin, Hexylcinnamal, Hexylsalicylat, Hydroxycitronellal, Hydroxyisohexyl 3-Cyclohexencarboxaldehyde, Isoeugenol, Lavendelöl, Lemonenöl, Limonen, Linalool, Linalylacetat, Linaylacetat, Mandarinenöl, Menthyl PCA, Methyl-2 Octynoat, Methylbenzoat, Methylcedrylketone, Methyldihydrojasmonate, Methylheptenon, Muskatnussöl, p-t-Butyl-alpha-methyldihydrocinnamic aldehyde, Rosmarinöl, süßes Orangenöl, Terpineol, Tonkabohnenöl, Triethylcitrat und/oder Vanillin zur Verwendung als Wespenabwehrmittel und zum Auftragen auf die Haut.

Insektenschutzmittel (Insektenvertreibungsmittel, Insektenabwehrmittel, Repellentien, Repellents) sind Präparate, die zur Abwehr und/oder Vertreibung von Insekten, aber auch Zecken und Milben äußerlich angewendet werden und verhindern sollen, dass diese auf der Haut aktiv werden. Insektenabwehrmittel sollen die Haut vor Belästigung durch Blut saugende oder beißende Insekten und andere Parasiten und/oder Lästlinge schützen, indem sie diese abwehren, bevor sie sich auf der Haut niederlassen. Die Mittel wirken dementsprechend nicht als Kontaktgifte, sondern nur als Abwehrmittel, da sie die Tiere nicht töten, sondern lediglich vertreiben.

Ein Repellent-Wirkstoff ist beispielsweise der 1-Piperidincarboxylsäure 2-(2-hydroxyethyl)-1-methylpropylester (INN: Icaridin, CAS-Nummer: 119515-38-7, Elincs-Nummer: 423-210-8), der die folgende Struktur aufweist:

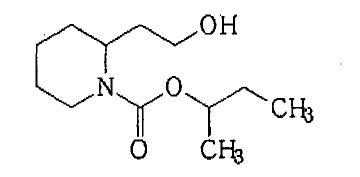

Ein weiterer eingesetzter Repellent-Wirkstoff ist der 3-(N-n-Butyl-N-acetyl-amino)propionsäureethylester (auch als Ethylbutylacetylaminopropionat oder Repellent 3535 bezeichnet), welcher sich durch die folgende Strukturformel auszeichnet:

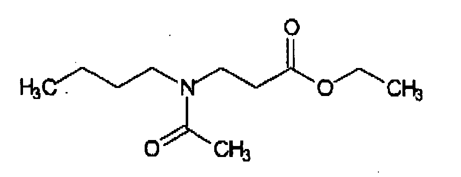

Nicht zuletzt kennt der Fachmann den Repellent-Wirkstoff N.N-Dlethyl-3-Methylbenzamid (Handelsbezeichnung: Meta-delphene, DEET), der die folgende Struktur aufweist:

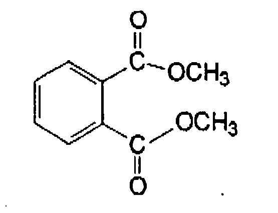

Diese Stoffe sind als Repellentwirkstoffe gegen Insekten, insbesondere Mücken oder Zecken, vielfach beschrieben, wie z.B. in DE 2246433, DE 19645250, DE 19645920.

In der WO 2006096876 und WO 2005034626 werden Dihydronepetalactone als Repellentien gegen Insekten offenbart. Der Offenbarungsgehalt zu Dihydronepetalacton der WO 2006096876 und WO 2005034626 sind hiermit explizit als erfindungsgemäß mitumfasst.

Katzenminze oder auch Nepeta ist als Mückenabwehrmittel bekannt und soll eine etwa zehn Mal stärkere Abwehr gegen Mücken als herkömmliche Abwehrmittel aufweisen.

In der DE 102005033845 wird u.a erwähnt bestimmte Partuminhaltsstoffe zur Abwehr von Mücken, Sandfliegen, Läusen, Bremsen, Wespen, Bienen, Fliegen und Zecken zu verwenden.

Problem ist dabei, dass ein Repellentwirkstoff, der beispielweise gegen Mücken abwehrend wirkt, diese Abwehrwirkung nicht oder nur eingeschränkt gegen andere Insekten aufweist.

Insbesondere haben sich die bekannten Insektenrepellentien als Abwehrmittel gegen Wespen bislang als ungeeignet erwiesen.

Zubereitungen mit einer hohen Konzentration an Repellentien können bei Menschen mit besonders empfindlicher Haut in Einzelfällen zu Hautirntationen führen. Ferner haben die Repellent-Wirkstoffe einen auch für die menschliche Nase wahrnehmbaren und auf Mitmenschen nicht gerade anziehend wirkenden Eigengeruch, der die Anwendung von Insektenabwehrmitteln nicht gerade attraktiv macht. Die abweisende Wirkung der Zubereitung erstreckt sich also nicht allein auf Insekten sondern darüber hinaus unter Umständen auch auf Mitmenschen, wenn diese über einen ausgeprägten Geruchsinn verfügen.

Wünschenswert ist es eine Zubereitung zur Verfügung zu stellen, die eine Abwehr von Wespen ermöglicht.
Es war eine weitere Aufgabe der vorliegenden Erfindung, ein Wespenabwehrmittel zu entwickeln, welches ein reduziertes Hautreizungspotential aufweist und welches einen für die menschliche Nase angenehm wohlriechenden Duft verströmt.

Die Erfindung umfasst eine Zubereitung enthaltend 3-(N-n-Butyl-N-acetyl-amino)propionsäureethylester. Dihydronepetalacton und/oder Extrakte der Katzenminze sowie ein oder mehrere Verbindungen gewählt aus der Gruppe der Parfuminhaltsstoffe 1,3,4,6,7,8-Hexahydro-4,6,6,7,8,8-hexamethylcyclopenta-gamma-2-benzopyran, 2,6-Dimethyl-7-octen-2-ol, 2-Acetonapthone-1,2,3,4,5,6,7,8-octahydro-2,3,8,8-tetramethyl, 2-Isobutyl-4-hydroxy-4-methyltetrahydropyran, 2-tert-Pentylcyclohexylacetat, 3,7-Dimethyl-2,6-octadien-1-ol, 3-Methyl-5-phenyl-1-pentanol, 7-Acetyl-1,1,3,4,4,6-hexamethyltetralin, Adipinsäurediester, alpha-Amylcinnamaldehyd, alpha-Hexylcinnamaldehyde, alpha-Isomethylionon, Alpha-Methylionon, Amyl C Butylphenylmethylpropionalcinnamal, Amylcinnamylalkohol, Amylsalicylat, Anisalkohol, Benzoin, Benzylacetat, Benzylalkohol. Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citral, Citronellol, Citronellylmethylcrotonat, Citronenöl, Coumarin, Diethylsuccinat, d-Limonene, Ethylenbrassylate, Ethyllinalool, Eugenol, Evernia Furfuracea Extract, Evernia Prunastri Extract, Farnesol, Geraniol, Guajakholzöl, Heliotropin, Hexylcinnamal, Hexylsalicylat, Hydroxycitronellal, Hydroxyisohexyl 3-Cyclohexencarboxaldehyde, Isoeugenol, Lavendelöl, Lemonenöl, Limonen, Linalool, Linalylacetat, Linaylacetat, Mandarinenöl, Menthyl PCA, Methyl-2 Octynoat, Methylbenzoat, Methylcedrylketone, Methyldihydrojasmonate, Methylheptenon, Muskatnussöl, p-t-Butyl-alpha-methyldlhydrocinnamic aldehyde, Rosmarinöl, süßes Orangenöl, Terpineol, Tonkabohnenöl, Triethylcitrat und/oder Vanillin zur Verwendung als Wespenabwehrmittel.

Die Zubereitung ist bevorzugt eine kosmetische Zubereitung und zum Auftragen auf die Haut geeignet.
3-(N-n-Butyi-N-acetyl-amino)propionsäureethylester wird nachfolgend entsprechend dem Handelsprodukt als IR 3535 bezeichnet.

Dihydronepetalactone sind beispielweise in der WO 2006096876 beschrieben, wobei hierin ein Einsatz als Wespenabwehr nicht offenbart wird.

Die Katzenminzen (Nepeta) bezeichnen eine Pflanzengattung der Familie der Lippenblütler (Lamiaceae). Erfindungsgemäß werden als Extrakte alle möglichen Extrakte, Lösungen oder Dispersionen der Nepetapflanze oder deren Teile verstanden.
Bei den in den Nepeta enthaltenen ätherischen Ölen handelt es sich in den Hauptbestandteilen um Citral, Citronellol, Geraniol und Limonen, sowie Nepetalacton, Gerb- und Bitterstoffe. Der Wirkstoff, der die Pflanze für Katzen so unwiderstehlich macht, ist Actinidin, ein iridoides Glykosid, das dem vergleichbaren Wirkstoff des Baldrian sehr ähnlich ist (BOWN, 1995). Die Nepeta enthält etwa 0,2-0,7 % ätherische Öle.

Der ölige Extrakt der Katzenminze, insbesondere aufgereinigt, ist wie jedes andere Naturöl sehr schlecht spreitend und sehr polar. Es ist deshalb unkosmetisch und verleiht den herkömmlichen kosmetischen Formeln eine ölig, fettige Phase. Es wird als schwer verteilbar wahrgenommen. Der polare Charakter des Extraktes führt zudem sehr schnell zu Instabilitäten, wie z.B. Öl- oder Wasserabscheidung.

Diesem Nachteil begegnet die vorliegende Erfindung ebenfalls.

Wespen möchten ihre Brut versorgen und brauchen daher Proteine und Zuckerreiches, um den eigenen Energiebedarf zu decken. Deshalb werden Wespen von Wurst und auch süßem Obst angelockt. Oft benutzen wir Menschen Parfum, die einen süßlichen Duft verströmen. Die Wespe findet uns attraktiv und landet auf der Haut, erst wenn sie nicht essbares findet, fliegt sie weiter. Aufgrund von Angst oder persönlichen Erlebnissen kommt es aber gar nicht dazu, sondern wir attackieren die anfliegende Wespe und versuchen sie zu verscheuchen.

In einem Test wurde nun gefunden, daß bestimmte Duftstoffe die Wespen nicht mehr anlocken. Es war überraschend, das die Duftstoffe aus der
Gruppe der Parfuminhaltsstoffe 1,3,4,6,7,8-Hexahydro-4,6,6,7,8,8-hexamethylcyclopenta-gamma-2-benzopyran, 2,6-Dimethyl-7-octen-2-ol, 2-Acetonapthone-1,2,3,4,5,6,7,8-octahydro-2,3.8,8-tetramethyl, 2-Isobutyl-4-hydroxy-4-methyltetrahydropyran, 2-tert-Pentyicyclohexylacetat, 3,7-Dimethyl-2,6-octadien-l-ol, 3-Methyl-5-phenyl-l-pentanol, 7-Acetyl-1,1,3,4,4,6-hexamethyltetralin, Adipinsäurediester, alpha-Amylcinnamaldehyd, alpha-Hexylcinnamaldehyde, alpha-Isomethyliorion, Alpha-Methylionon, Amyl C Butylphenylmethylpropionalcinnamal, Amylcinnamylalkohol, Amylsalicylat, Anisalkohol, Benzoin, Benzylacetat, Benzylalkohol, Benzylbenzoat, Benzylcinnamat, Benzylsalicylat. Bergamotöl, bitteres Orangenöl, Butylphenylmethylpropioal, Cardamomöl. Cedrol, Cinnamal, Cinnamylalkohol, Citral, Citronellol, Citronellylmethylcrotonat, Citronenöl, Coumarin, Diethylsuccinat, d-Limonene, Ethylenbrassylate, Ethyllinalool, Eugenol, Evemia Furfuracea Extract, Evemia Prunastri Extract, Famesol, Geraniol, Guajakholzöl, Heliotropin, Hexylcinnamal, Hexylsalicylat, Hydroxycitronellal, Hydroxyisohexyl 3-Cyclohexencarboxaldehyde, Isoeugenol, Lavendelöl, Lemonenöl, Limonen, Linalool, Linalylacetat, Linaylacetat, Mandarinenöl, Menthyl PCA, Methyl-2 Octynoat, Methylbenzoat, Methylcedrylketone, Methyldihydrojasmonate, Methylheptenon. Muskatnussöl, p-t-Butyl-alpha-methyldihydrocinnamic aldehyde, Rosmarinöl, süßes Orangenöl, Terpineol, Tonkabohnenöl, Triethylcitrat und/oder Vanillin die Wespen nicht mehr anlockt,
Wird diese Zubereitung in Form eines Geles auf die Haut aufgetragen, so gibt sich eine Langzeit-Effekt.

In der Gelmatrix lassen sich die Repellentien und Parfumbestandteile stabil formulieren und werden nur langsam an die Umwelt abgegeben.

Bevorzugt ist eine erfindungsgemäße Zubereitung mit weiteren Insektenrepellentien, wie z.B. 1-Piperidincarboxylsäure 2-(2-hydroxyethyl)-1-methylpropylester (INN: lcaridin), Ethylbutylacetylaminopropionat (EBAAP) und/oder N,N-Diethyl-3-methylbenzamid (DEET) zu kombinieren.

Es ist erfindungsgemäß vorteilhaft, wenn IR 3535, Dihydronepetalacton und/oder Extrakte der Katzenminze in einer Gesamtkonzentration von 0,01 bis 40 Gew.%, jeweils bezogen auf das Gesamtgewicht der Zubereitung, in dieser enthalten sind.

Die erfindungsgemäßen Parfuminhaltstoffe, ein oder mehrere, sind bevorzugt zu einer Gesamtkonzentration von 1 ppm bis 2,5 Gew.%, jeweils bezogen auf das Gesamtgewicht der Zubereitung, in dieser enthalten.

Dabei ist es erfindungsgemäß bevorzugt, wenn als Parfuminhaltsstoff eine Verbindungen aus der Gruppe Hexylsalicylat, Linaylacetat oder 2-Isobutyl-4-hydroxy-4-methyltetrahydropyran eingesetzt werden.

Eine vorteilhafte Ausführungsform im Sinne der vorliegenden Erfindung ist eine kosmetische Zubereitung als Hydrodispersionsgel enthaltend 15 Gew.% IR 3535 und 0,004 Gew.% Hexylsalicylat, jeweils bezogen auf das Gesamtgewicht der Zubereitung.

Die erfindungsgemäße kosmetische Zubereitung ist vorteilhaft dadurch gekennzeichnet, das sie in Form einer wässrigen oder wässrig-alkoholischen Lösung, einer Emulsion (W/O, O/W, WIS, S/W oder multiple Emulsion, Makro- oder Mikroemulsion), einer Dispersion, einer Pickering-Emulsion, eines Gels, eines Hydrodispersionsgels oder einer wasserfreien Zubereitung vorliegt. Dabei ist es erfindungsgemäß bevorzugt, wenn die kosmetische Zubereitung in Form eines Hydrodispersionsgels Emulsion vorliegt.

Die Zubereitung kann erfindungsgemäß vorteilhaft in Form einer dünnflüssigen, sprühfähigen wässrigen oder wässrig-alkoholischen Lösung, in Form eines Gels, als Salbe, Creme oder Lotion (ggf. sprühbar) vorliegen.

Die Zubereitung kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Zubereitung, erfindungsgemäß.

Die Wasserphase der erfindungsgemäßen Zubereitung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmoriomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Eine eventuell vorhandene Ölphase kann alle herkömmlichen Bestandteile von in der Kosmetik eingesetzten Öl-, Fett- und Wachskomponenten enthalten.

Als Emulgatoren können alle aus der Kosmetik bekannten Emulgatoren und Emulgatorsysteme eingesetzt werden.

Neben der erfindungsgemäßen Wirkstoff-Kombination kann die erfindungsgemäße Zubereitung weitere kosmetische Wirk- und Pflegestoffe enthalten, beispielsweise die nach der Kosmetikverordnung zugelassenen UV-Lichtschutzfilter, und Konservierungsmittel bzw.

Konservierungshelfer. Derartige Wirkstoffe können erfindungsgemäß vorteilhaft in Konzentrationen von 0,01 bis 30 Gew-%. bezogen auf das Gesamtgewicht der Zubereitung in dieser enthalten sein.

Als weitere Pflegestoffe können insbesondere Niacinamid, Panthenol, Aloe vera. Hammamelis-Extrakt, Polidocanol, Vitamin E, Vitamin E-Derivate. Vitamin A, Vitamin A-Derivate, Vitamin C, Vitamin C-Derivate, Coenzym Q10, Kreatin, Taurin, Alpha-Glycosylrutin in Konzentrationen von 0,1 bis 30 Gewichts-%, bezogen auf das Gesamtgewicht der Zubereitung eingesetzt werden.

Erfindungsgemäß besonders bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gewichts-%, bezogen auf das Gesamtgewicht der Zubereitung eingesetzt.

Neben der Wespenabwehr sind aber vor allem die Hautverträglichkeit und das Hautgefühl entscheidende Eigenschaften eines topisch zu applizierenden insbesondere kosmetischen Produktes.

Es nützt eine hervorragende Repellentwirksamkeit nichts, wenn das Kosmetikprodukt zu Unverträglichkeiten führt.
Eine der häufig auftretenden Hautunverträgfichkeiten ist der sog. Stiriging-Effekt, der sich insbesondere im Gesicht unangenehm bemerkbar macht. Der Stinging-Effekt tritt insbesondere im Nasolabial-Bereich auf und, bedeutet ein subjektives Missempfinden der Probanden.
Um solche unerwünschten Effekte für Kosmetika möglichst auszuschliessen werden vor Produkteinführung sog. Stinging-Tests durchgeführt. Jeweils zwei Produkte werden dabei im dirketen Paarvergleich auf ihre Hautverträglichkeit getestet. Als interner High-Standard (Benchmark) wird dabei ein als hautverträglich bekanntes handelsübliches Produkt verglichen (NIVEA Visage Day Cream normal mixed).

In zahlreichen Untersuchungen wurde nun festgestellt, dass ein wesentlicher Beitrag zum Stinging durch die in vielen Kosmetika enthaltenen Parabene hervorgerufen wird, insbesondere wenn diese mit einem Anteil von mehr als 0,3 Gew.% in der Rezeptur vorliegen.
Parabene werden als Konservierungsmittel eingesetzt, so dass deren Ersatz mitunter schwierig ist.

Es wurde nun überaschenderweise fest gestellt, dass die erfindungsgemäßen Zubereitungen sehr gute Wespenabwehrwirksamkeiten zeigen aber einen Stinging-Effekt nicht aufweisen. Die erfindungsgemäßen Zubereitungen lassen sich daher bevorzugt mit einem Anteil von bis zu 0,3 Gew.% Konservierungsmittel, wie insbesondere Parabene, herstellen.
Bevorzugt kann auf den Zusatz von Konservierungsmitteln, wie z.B. Parabenen, ganz verzichtet werden.
D.h es ist erstmals möglich Repellenthaltige Zubereitungen zur Verfügung zu stellen, die gleichzeitig verbesserte Verträglichkeit aufweisen und insbesondere keine Stinging-Effekte aufweisen.
Die erfindungsgemäßen Zubereitungen sind daher bevorzugt frei von bakteriziden-, fungiziden-, sporicidally d.h. sporenabtötenden, wirksamen Mitteln und/oder frei von Konservierungsmitteln, Weiterhin sind sie bevorzugt frei von Formaldehyd, Formaldehyddonatoren wie Diazolidinyl urea, Imidazolidinyl urea und/oder DMDM Hydantoin sowie insbesondere frei von Benzoesäure, p-Hydroxybenzoesäure und/oder oder deren Derivate oder Salze. Bevorzugt umfassen die erfindungsgemäßen Zubereitungen weniger als 0,3 Gew.% bzw. insbesondere sind sie frei von Parabenen, insbesondere frei von Methylparaben, Ethylparaben, Propylparaben, Isopropylparaben, Butylparaben, Isobutylparaben und/oder Benzylparaben. Auch 2,4-Dichlorobenzylalkohol, 4-Chloro-3,5-dimethyl-phenol, 2-Bromo-2-nitropropane-1,3-diol und/oder lodopropinylbutylcarbamate (IPBC) sowie Quaternium-15 (CAS. 51229-78-8), Methyl-chloroisothiazolinone und/oder Methylisothiazolinon sind in den erfindungsgemäßen Zubereitungen nicht bzw. zu weniger als 0,3 Gew.% zu finden.
Die erfindungsgemäßen Zubereitungen sind besonders bevorzugt Parabenfrei.
Es bedeutet erfindungsgemäß "frei von" ein Anteil von weniger als 0,1 Gew.%, bezogen auf die Gesamtmasse der Zubereitung. Bevorzugt beträgt der Anteil an den zuvor genannten Mitteln und insbesondere Parabenen 0 Gew.%.

Als insbesondere kosmetische Zubereitung ist die erfindungsgemäße Zubereitung als topisch anzuwendendes Mittel vorteilhaft geeignet.

Aber auch als Zubereitung, aufgebracht auf Gegenstände und Kleidung oder versprüht in der Luft, zeigt sie eine eindeutig wespenabwehrende Wirkung.

Erfindungsgemäß ist insbesondere die Verwendung der erfindungsgemäßen Zubereitung zur Prophylaxe von Wespenstichen.

In einer Untersuchung wurde die erfindungsgemäße Zubereitung auf deren Verwendung als Wespenabwehrmittel getestet.
Die erfindungsgemäße Rezeptur (Netero 663) umfasst

| INCl | Netero 663 |
|---|---|
| Aqua | 53,275 |
| Cyclomethicone | 8 |
| Glycerin | 9 |
| Sodium Hydroxide | 0,2 |
| Alcohol Denat. | 10 |
| Ethyl Butylacetylaminopropionate | 15 |
| Acrylates/C10-30 Alkyl Acrylate Crosspolymer | 0,2 |
| Carbomer | 0,2 |
| Parfum* | 0,2 |
| Cl 42090 | 0,4 |
| Aloe Barbadensis | 0,025 |
| Chondrus Crispus | 0,2 |
| Hamamelis Virginiana Distillate | 0,1 |
| Sodium Polyacrylate | 0,2 |
| Cyclomethicone + Dimethiconol | 1 |
| Methylpropanediol | 2 |

| | |
|---|---|
| * Parfum enthält: 34,5% Limonen und 6,5% Linalool | |

Die Versuche erfolgten mit je zwei Substanzen an zwei aufeinanderfolgenden Tagen. Jeweils 6 Personen nahmen am Test mit einer Substanz teil. In vier übliche Fliegenkäfige mit einem Arm-Einlaß und Abschluß aus Baumwolle wurden vor dem Versuch je 20 frisch gefangene, voll aktive Wespen verbracht *(Paravespula vulgaris).* Die linke Hand und der linke Arm jedes Probanden wurden unmittelbar vor dem Versuch oben und unten mit der jeweiligen Testsubstanz eingerieben bzw. besprüht. Dann wurde die Substanz kurz (etwa 1-2 Minuten einziehen gelassen). An der Oberfläche des Unterarms wurde dann etwas oberhalb des Handgelenks an beiden Armen ein Tropfen Waldhonig angebracht. Dieser Waldhonig hatte sich in Vorversuchen als überaus attraktiv für diese Wespenart erwiesen. Danach steckte jeder Proband seinen rechten, unbehandelten Arm als Kontrolle in den Käfig hinein. Diese unbehandeltes Armareal wurde als Kontrolle beobachtet.

Es wurde die Zeit erfaßt, bis mindestens 3 Wespen sich dauerhaft auf dem Honig niedergelassen hatten und daran fraßen. Diese Zeiten wurden protokolliert. Als Ausschlusszeit wurde . 2 Minuten gewählt. Unmittelbar danach steckte jeder Proband den besprühten Arm in den Käfig, und es wurde wiederum festgestellt, wie lange es dauert, bis drei Wespen fraßen. Dies wurde nach einer Stunde wiederholt - ohne jedoch neue Repellent Substanz auf die Haut zu sprühen. Dies wurde nach 2 Stunden nochmals wiederholt.

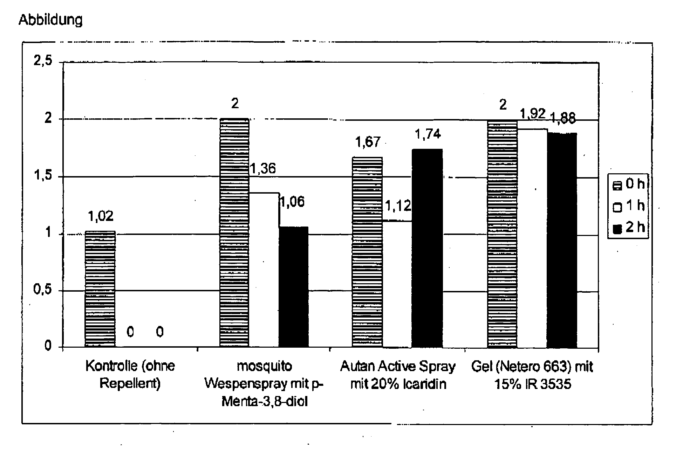

Abbildung 1 zeigt die Testergebnisse, die belegen, dass die erfindungsgemäße Zubereitung eine erhöhte Wespenabwehr zeigt als die bekannten insektenabwehrmittel (Autan, Mosquito). Nach durchschnittlich 2 Minuten (Ausschlußfrist) hatte sich auf dem Arm mit der erfindungsgemäßen Zubereitung keine Wespe niedergelassen. Auf dem Arm mit Autan setzten sich nach einer Stunde innerhalb von 1,12 Minuten schon 3 Wespen. Nach 2 h war der Wert schon etwas besser, allerdings reichte der Schutz zu keinem Zeitpunkt an Netero 633 heran. Diese Zubereitung mit 15% IR 3535 zeigte zu allen Zeitpunkten, den besten Schutz vor Wespen.

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

### W/O-Emulsionen

| | **1** | **2** | **3** | **4** | **5** |
|---|---|---|---|---|---|
| Triglycerindüsostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 17,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0.25 |
| Octyldodecanol | 0.5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Iminodibernsteinsäure | 0,5 | --- | --- | --- | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| 3-(N-n-Butyl-N-acetyl-amino)propionsäureethylester | 5 | 15 | 8 | 10 | 3 |
| Hexylsalicylat | 0,3 | 0,4 | 0,25 | 0,15 | 150 ppm |
| Ethanol | 2,0 | --- | 5,0 | --- | --- |
| Capryl-/Caprinsäureiriglycerid | 2,0 | 2,5 | 3,0 | 5,0 | 0,5 |
| Methylparaben | 0,4 | 0.15 | 0,05 | 0,3 | 0,4 |
| Propylparaben | 0,3 | 0,4 | 0,25 | 0,15 | --- |
| Fucose | 0,5 | 1,5 | 1 | 0,5 | 0,1 |
| Raffinose | 0,5 | 0,75 | 1 | 0,35 | 0,1 |
| Galactose | 0,5 | 0,35 | 1 | 1,75 | 0,1 |
| Wasser | ad 100 | ad 100 | ad 100 | ad 100 | ad 100 |

### W/O-Emuisionen

| | **6** | **7** | **8** | **9** | **10** |
|---|---|---|---|---|---|
| PEG-30 Dipolyhydroxystearat | --- | 0,5 | 0,25 | --- | 3,0 |
| Lanolin Alcohol | 1,0 | 1,5 | 1,75 | 3,0 | --- |
| Paraffinöl | 12.5 | 10.0 | 8,0 | 5,0 | 17,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Aluminiumstearat | 0.4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| 3-(N-n-Butyl-N-acetyl-amino)propionsäureethylester | 12,5 | 1,0 | 7,5 | 10 | 5 |
| Magnesiumsulfat | 0,5 | 0,6 | 0,5 | 0.7 | 1,0 |
| Glycerin | --- | 5,0 | --- | 15,0 | 5,5 |
| Tetranatriumiminosuccinat | 1,5 | 0,6 | 3,0 | 0,4 | 1,0 |
| Linaylcetat | 0,15 | 15 ppm | 0,5 | 0,0025 | 0,0065 |
| 1,3 Butylenglykol | --- | --- | 5,0 | --- | 7,5 |
| Capryl-/Caprinsäuretriglycerid | 2,0 | 2,5 | 3,0 | 5,0 | 0,5 |
| Methylparaben | 0,4 | 0,15 | 0,05 | 0,3 | 0,4 |
| Mannose | 0,5 | 1,5 | 1 | 0,5 | 0,1 |
| Rhamnose | 0,75 | 0,75 | 1,25 | 0,35 | 0,1 |
| Fucose | 1,5 | 0,35 | 1 | 1,75 | 0,1 |
| Propylparaben | 0,3 | 0,4 | 0,25 | 0,15 | |
| Wasser | ad 100 | ad 100 | ad 100 | ad 100 | ad 100 |

### W/S-Emulsion

| | **11** | **12** | **13** | **14** | **15** |
|---|---|---|---|---|---|
| Cetyldimethicanecopolyol | 1,0 | --- | --- | 3,0 | 5,0 |
| Cylomethicon + Dimethiconcopolyol | 10,0 | 12,5 | 25 | --- | --- |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75. | 0,25 | 0,1 |
| Magnesiumchlorid | 2.0 | 0,6 | 2,5 | 0,7 | 1,0 |
| Glycerin | 25,0 | 5,0 | 10,0 | 15,0 | 57,5 |
| 3-(N-n-Butyl-N-acetyl-amino)propionsäureethylester | 6 | 10 | 12 | 1,5 | 5,0 |
| Hexylsalicylat | 0,3 | 0,04 | 0,25 | 0,12 | 150 ppm |
| Methylparaben | 0,4 | 0,1 | 0,05 | 0,3 | 0,4 |
| Butylenglykol | --- | 5,0 | --- | --- | 7.5 |
| Propylparaben | 0,3 | 0,4 | 0,25 | 0,15 | --- |
| Cetyldimethicon | 0,5 | --- | 0.7 | --- | --- |
| lodopropynylbutylcarbamat | --- | --- | 0,05 | --- | 0,1 |
| Fucose | 0,5 | 1,5 | 1 | 0,5 | 0,1 |
| Raffinose | 0,5 | 0,75 | 1 | 0,35 | 0,1 |
| Galactose | 0,5 | 0,35 | 1 | 1,75 | 0,1 |
| modifizierte Stärke | --- | 2,5 | --- | 0,15 | --- |
| Wasser | ad 100 | ad 100 | ad 100 | ad 100 | ad 100 |

### O/W-Emulsionen

| | **16** | **17** | **18** | **19** | **20** |
|---|---|---|---|---|---|
| Glycerylstearatcitrat | 2 | --- | --- | --- | --- |
| Glycerylsterat | --- | 5 | 2 | 3 | --- |
| PEG-40-Stearat | --- | --- | 1 | --- | --- |
| Triglycerinmethylglucosedistearat | --- | --- | --- | --- | 3 |
| Sorbitanstearat | --- | --- | --- | --- | 1 |
| Cetearylglucosid | --- | --- | --- | 2 | --- |
| Behenylalkohol | --- | --- | --- | --- | 1 |
| Stearylalkohol | 2 | 1 | --- | --- | --- |
| Cetylstearylalkohol | --- | --- | 2 | --- | --- |
| Hydrierte Cocosfettglyceride | 2 | --- | --- | 1 | --- |
| Sheabutter | --- | 2 | --- | --- | --- |
| Butylenglycol Dicaprylat/Dicaprat | 1 | --- | --- | --- | --- |
| Caprylic/Capric Triglyceride | --- | 4 | --- | --- | 1 |
| Ethylhexylkokosfettsäureester | 3 | --- | --- | --- | --- |
| Octyldodecanol | --- | --- | 5 | 8 | --- |
| Mineralöl | 8 | 1 | --- | --- | 5 |
| 3-(N-n-Butyl-N-acetyl-amirio)propionsäureethylester | 8 | 3 | 10 | 12 | 7,5 |
| Vaseline | 4 | --- | --- | 2 | --- |
| Octamethyltetrasiloxan | 5 | 1 | 3 | 1 | 3 |
| Dimethylpolysiloxan | --- | 3 | 1 | 3 | 2 |
| Dicarprylylcarbonat | 10 | 1 | 8 | 5 | 2 |
| Glycerin | 3,0 | --- | 25 | 12,5 | 30 |
| Methylparaben | 0,3 | --- | --- | 0,2 | 0,4 |
| Galactose | 0,15 | 0,1 | 1 | 1,5 | 0,25 |
| N-Acetylglucosamin | 0,3 | 0,1 | 1,25 | 2,5 | 0,75 |
| Fucose | 1,5 | 0,1 | 3 | 0,75 | 0,25 |
| lodopropynylbutylcarbamat | 0,1 | 0,2 | 0,2 | 0,05 | --- |
| Linaylcetat | 0,15 | 15 ppm | 0,5 | 0,0025 | 0,0065 |
| Wasser | Ad 100 | Ad 100 | Ad 100 | Ad 100 | Ad 100 |

### O/W-Emulsionen

| | **21** | **22** | **23** | **24** | **25** |
|---|---|---|---|---|---|
| Glycerylstearatcitrat | 5 | --- | --- | --- | --- |
| Glycerylstearat | --- | 5 | --- | --- | --- |
| PEG-40-Stearat | --- | 2 | --- | --- | --- |
| Polyethylenglycol(21)stearylether | --- | --- | 2 | --- | --- |
| Polyethylenglycol(2)stearylether | --- | --- | 1 | --- | --- |
| Cetearylglucosid | --- | --- | --- | 2 | --- |
| Stearinsäure | --- | --- | --- | --- | 2,5 |
| Behenylalkohol | --- | --- | --- | --- | 2 |
| Stearylalkohol | 2 | 1 | --- | 5 | --- |
| Cetylstearylalkohol | --- | --- | 2 | --- | 1 |
| Hydrierte Cocosfettglyceride | 2 | --- | --- | 3 | 1 |
| Butylenglycol Dicaprylat/Dicaprat | 1 | --- | 8 | --- | 2 |
| Caprylic/Capric Triglyceride | --- | 4 | 2 | --- | --- |
| Ethylhexylkokosfettsäureester | 3 | 6 | --- | --- | 2 |
| Octyldodecanol | --- | --- | 1 | 9 | --- |
| Mineralöl | 9 | 1 | 1 | 1 | 3 |
| Vaseline | 4 | 2 | 5 | 0,5 | 0.75 |
| Glycerin | 7,5 | 15 | 65 | 25 | --- |
| 3-(N-n-Butyl-N-acetyl-amino)propionsäureethyester | 3,5 | 3 | 8,5 | 25 | 15 |
| Raffinose | 0,15 | 0,1 | 1 | 1,5 | 0,25 |
| N-Acetylglucosamin | 0,3 | 0,1 | 1,25 | 2,5 | 0,75 |
| Fucose | 1,5 | 0,1 | 3 | 0,75 | 0,25 |
| Octamethyltetrasiloxan | --- | 1 | 2 | 5 | --- |
| Dimethylpolysiloxan | 0,5 | 0,75 | 1,25 | --- | 1 |
| Dicarprylylcarbonat | 6 | 2 | 10 | 0,5 | 4 |
| Methylparaben | 0,3 | --- | 0,1 | --- | 0.05 |
| 2-Isobutyl-4-hydroxy-4-methyltetrahydropyran | 0,3 | 60 ppm | 0,0125 | 0,065 | 0,75 |
| lodopropynylbutylcarbamat | 0,1 | 0.2 | 0,1 | 0,2 | 0,15 |
| Wasser | Ad 100 | Ad 100 | Ad 100 | Ad 100 | Ad 100 |

### Hydrodispersionsgel

| | **26** | **27** | **28** | **29** | **30** |
|---|---|---|---|---|---|
| Silikonöl, cyclisch | 8 | 10 | 5 | 3 | --- |
| Silikonöl, linear | --- | --- | --- | --- | 3 |
| Dimethiconol | 1 | 2 | 3 | --- | 3 |
| Ethanol | 10 | 15 | 7,5 | 5 | 3 |
| 3-(N-n-Butyl-N-acetyl-amino)propionsäureethylester | 15 | 5 | 15 | 20 | 30 |
| Natriumpolyacrylat | 0,2 | 0,3 | 0,3 | 0,4 | 0,10 |
| Methylpropandiol | 2 | 3 | 4 | 5 | |
| Glycerin | 9 | 15 | 5 | 7,5 | 25 |
| Carbomer | 0,2 | 0,3 | 0,2 | 0,4 | 0,15 |
| Acrylates/C10-30Alkyl Acrylate Crosspolymer | 0,2 | 0,15 | 0,3 | 0,4 | 0,10 |
| Carrageenan (Chondrus Crispus) | --- | --- | --- | --- | 2 |
| Hamamelis Extrakt | 0,1 | 0,2 | 0,3 | 0,4 | --- |
| 2-Isabutyl-4-hydraxy-4-methyltetrahydrdpyran | 0,3 | 60 ppm | 0,0125 | 0,065 | 0,75 |
| Parfum | q.s. | q.s. | q.s. | q.s. | q.s. |
| Farbstoff | q.s. | q.s. | q.s. | q.s. | q.s. |
| Wasser | ad 100 | ad 100 | ad 100 | ad 100 | ad 100 |
| Aloe Vera Extrakt | 0,1 | 0,2 | 0,5 | 0,2 | 1,0 |

### ethanolisches Spray

| | **31** | **32** | **33** | **34** | **35** |
|---|---|---|---|---|---|
| Glycerin | 9 | 15 | 20 | 5 | 7.5 |
| Ethanol | 42,5 | 45 | 40 | 35 | 25 |
| 3-(N-n-Butyl-N-acetyl-amino)propionsäureethylester | 15 | 10 | 5 | 20 | 25 |
| Wasser | ad 100 | ad 100 | ad 100 | ad 100 | ad 100 |
| PPG-1-PEG-9-Laurylglycolether | 0,4 | 0,2 | 0,6 | 1,0 | 5 |
| Methylheptenone | 0,3 | 0,025 | 0,125 | 150 ppm | 0,75 |
| Hamamelis Extrakt | 0,1 | 0,2 | 0.35 | 0,4 | --- |
| Aloe Vera Extrakt | 0,025 | 0,2 | 0,5 | 0,02 | 1,0 |

### O/W-Emulsionen, leicht

| | **36** | **37** | **38** | **39** | **40** |
|---|---|---|---|---|---|
| 3-(N-n-Butyl-N-acetyl-amino)propionsäureethylester | 15 | 10 | 5 | 20 | 30 |
| Glycerylstearat | 1 | 1 | 2 | 5 | 2 |
| Cetearylalkohol + Natriumcetearylsulfat | 3 | 4 | 5 | 2 | 8 |
| Phenoxyethanol | 0,5 | 0,6 | 0,75 | 1,0 | 0.35 |
| Xanthan Gummi | 0,5 | 0,75 | 0,35 | 0,4 | 1,0 |
| Menthyl PCA | 0,5 | 2,5 | 1,5 | 2,0 | 0,25 |
| Hamamelis Extrakt | 0,1 | 0,2 | 0,35 | 0,4 | --- |
| Aloe Vera Extrakt | 0,025 | 0,2 | 0,5 | 0,02 | 1,0 |
| Dicaprylylcarbonat | 5 | 7,5 | 3 | 10 | 2,5 |
| Caprylic/Capric Trilycerid | 5 | 7,5 | 3 | 2,1 | 1,8 |
| Silikonöl, linear | 3 | 2 | --- | 0,75 | 5 |
| Caprylylglykol | 0,5 | --- | 0,3 | --- | 0,1 |
| Ethanol | 2,5 | 3,5 | 5 | 1,5 | --- |

## Patentansprüche

1. Verwendung einer Zubereitung umfassend 3-(N-n-Butyl-N-acetyl-amino)propionsäureethylester, Dihydronepetalacton und/oder ein Extrakt aus der Katzenminze sowie ein oder mehrere Verbindungen gewählt aus der Gruppe der Parfuminhaltsstoffe 1,3,4,6,7,8-Hexahydro-4,6,6,7,8,8-hexamethyleyclopenta-gamma-2-benzopyran, 2,6-Dimethyl-7-octen-2-ol, 2-Acetonapthone-1,2,3,4,5,6,7,8-octahydro-2,3,8,8-tetramethyl, 2-Isobutyl-4-hydroxy-4-methyltetrahydropyran, 2-tert-Pentylcyclohexylacetat, 3,7-Dimethyl-2,6-octadien-1-ol, 3-Methyl-5-phenyl-1-pentanol, 7-Acetyl-1,1,3,4,4,6-hexamethyltetralin, Adipinsäurediester, alpha-Amylcinnamaldehyd, alpha-Hexylcinnamaldehyde, alpha-Isomethylionon, Alpha-Methylionon, Amyl C Butylphenylmethylpropionalcinnamal, Amylcinnamylalkohol, Amylsalicylat, Anisalkohol, Benzoin, Benzylacetat, Benzylalkohol, Benzylbenzoat, Benzylcinnamat, Benzylsalicylat, Bergamotöl, bitteres Orangenöl, Butylphenylmethylpropioal, Cardamomöl, Cedrol. Cinnamal, Cinnamylalkohol, Citral, Citronellol, Citronellylmethylcrotonat, Citronenöl, Coumarin, Diethylsuccinat, d-Limonene, Ethylenbrassylate, Ethyllinalool, Eugenol, Evemia Furfuracea Extract, Evernia Prunastri Extract, Farnesol, Geraniol, Guajakholzöl, Heliotropin, Hexylcinnamal, Hexylsalicylat, Hydroxycitronellal, Hydroxyisohexyl 3-Cyclohexencarboxaldehyde, Isoeugenol, Lavendelöl, Lemonenöl, Limonen, Linalool, Linalylacetat, Linaylacetat, Mandarinenöl, Menthyl PCA, Methyl-2 Octynoat, Methylbenzoat, Methylcedrylketone, Methyldihydrojasmonate, Methylheptenon, Muskatnussöl, p-t-Butyl-alpha-methyldihydrocinnamic aldehyde, Rosmarinöl, süßes Orangenöl, Terpineol, Tonkabohnenöl, Triethylcitrat und/oder Vanillin als Wespenabwehrmittel.

2. Verwendung einer Zubereitung nach Anspruch 1 indem der Parfumstoff gewählt wird aus der Gruppe Hexylsalicylat, Linaylacetat und/oder 2-Isobutyl-4-hydroxy-4-methyltetrahydropyran.

3. Verwendung einer Zubereitung nach einem der vorstehenden Ansprüche indem 3-(N-n-Butyl-N-acetyl-amino)propionsäureethylester gewählt wird.

4. Verwendung einer Zubereitung nach einem der vorstehenden Ansprüche zum Auftragen auf die Haut.

5. Verwendung einer Zubereitung nach einem der vorstehenden Ansprüche umfassend weitere Insektenrepellentien, insbesondere 1-Piperidincarboxylsäure 2-(2-hydroxyethyl)-1-methylpropylester (INN: Icaridin), Ethylbutylacetylaminopropionat (EBAAP) und/oder N,N-Diethyl-3-methylbenaamid (DEET).

6. Verwendung nach einem der vorstehenden Ansprüche, **dadurch gekennzeichnet, dass** die Zubereitung maximal 0,3 Gew.% bakterizide-, fungizide-, sporicidally wirksame Mittel, Konservierungsmittel und/oder Formaldehyd und Formaldehyddonatoren enthält.

7. Verwendung nach einem der vorstehenden Ansprüche, **dadurch gekennzeichnet, dass** die Zubereitung frei von bakteriziden-, fungiziden-, sporicidally wirksamen Mitteln, Konservierungsmitteln und/oder Formaldehyd und Formaldehyddonatoren ist.

8. Verwendung nach einem der vorstehenden Ansprüche, **dadurch gekennzeichnet, dass** die Zubereitung frei von Parabenen ist.

9. Verwendung einer Zubereitung nach einem der vorstehenden Ansprüche, **dadurch gekennzeichnet, dass** die kosmetische Zubereitung in Form einer Emulsion oder Hydrodispersionsgel vorliegt.

10. Verwendung einer Zubereitung nach einem der vorstehenden Ansprüche, **dadurch gekennzeichnet, dass** die Zubereitung als Tränkungsmedium auf Vlies, Pflaster oder Tuch aufgetragen wird.
